# EUROPÄISCHE PATENTSCHRIFT

(11) **EP 0 854 148 B1**
(45) Veröffentlichungstag und Bekanntmachung des Hinweises auf die Patenterteilung: **02.05.2002**
(21) Anmeldenummer: 97123039.6
(22) Anmeldetag: 31.12.1997
(51) Int. Cl.: C07H 1/00, C07H 15/04

(54) **Verfahren zur Hydrierung von Zuckern**
Process for the hydrogenation of sugars
Procédé d'hydrogénation de sucres

(30) Priorität: 17.01.1997 DE 19701439
(43) Veröffentlichungstag der Anmeldung: 22.07.1998
(73) Patentinhaber: SÜDZUCKER AKTIENGESELLSCHAFT MANNHEIM/OCHSENFURT, 68165 Mannheim (DE)
(72) Erfinder: Degelmann, Hanspeter, 67549 Worms (DE); Kowalczyk, Jörg, Dr., 67248 Bockenheim (DE); Kunz, Markwart, Dr., 67550 Worms (DE); Schüttenhelm, Matthias, Dr., 67550 Worms (DE)
(74) Vertreter: Schrell, Andreas, Dr.

(56) Entgegenhaltungen:
- DE-A- 2 362 552
- DE-A- 19 523 008
- GB-A- 801 732
- US-A- 3 965 199

## Beschreibung

Die vorliegende Erfindung betrifft ein Verfahren zur Hydrierung von Zuckern oder Zuckergemischen aus der Gruppe bestehend aus Isomaltulose, Trehalulose, Maltulose, Leucrose und Lactulose zu den entsprechenden Zuckeralkoholen oder Zuckeralkoholgemischen, wobei die Zucker oder Zuckergemische in wässriger Lösung unter erhöhter Temperatur und erhöhtem Druck mit Wasserstoff unter Verwendung eines trägergebundenen Nickel-haltigen Katalysators hydriert werden.

Aus der EP 0 152 779 B1 ist ein Verfahren zur Herstellung eines Gemisches von 1-0-α-D-Glucopyranosyl-D-mannit (im folgenden 1,1-GPM genannt) und 6-0-α-D-Glucopyranosyl-D-sorbit (im folgenden 1,6-GPS genannt) aus 6-0-α-D-Glucopyranosyl-Dfructose (Isomaltulose, Palatinose^{R}) bekannt. Gemäß dem beschriebenen Verfahren wird Isomaltulose bei erhöhtem Druck und erhöhter Temperatur kontinuierlich im Festbettverfahren hydriert, wobei trägerfreie Katalysatoren aus Elementen der achten Nebengruppe des Periodensystems, insbesondere Nickel, Kobalt und Eisen, verwendet werden. Das Produkt der beschriebenen Verfahrensweise weist 1,6-GPS und 1,1-GPM in einem Verhältnis von ca. 1:1 auf.

Aus der DE 44 16 115 A1 ist ebenfalls ein Verfahren zur Herstellung von 1,6-GPS und 1,1-GPM aus Isomaltulose bekannt, wobei der in der EP 0 152 779 B1 beschriebene Katalysator zusätzlich Elemente der sechsten Nebengruppe des Periodensystems enthält. Auch in diesem Fall wurde eine Produktzusammensetzung von 1,6-GPS zu 1,1-GPM von ca. 1:1 erhalten. Die DE 44 16 408 A1 und die DE 39 34 457 A1 beschreiben ebenfalls Verfahren zur Hydrierung von Zuckern, beispielsweise von Glucose, Xylose, Lactulose oder Maltose. Die für die Hydrierungsreaktion verwendeten Katalysatoren sind trägerfreie Formkörper aus Elementen der achten und sechsten Nebengruppe des Periodensystems.

Aus der DE 195 23 008 A1 ist ein Verfahren zur Herstellung von Isomaltit durch Hydrierung einer Isomaltulose-Lösung bekannt, wobei die Konzentration von Isomaltulose im Bereich von 20 bis 50 Gew.-% liegt. Die Hydrierung erfolgt bei einer Temperatur von 80°C bis 130°C unter Verwendung eines Katalysators, der aus Ruthenium, Nickel und deren Mischungen auf einem inerten Träger besteht, wobei der ausgeübte Druck weniger als 50 Atmosphären beträgt.

Aus der DE 2 362 552 A ist ein Verfahren zur katalytischen Hydrierung von Zuckern, beispielsweise Invertzucker, Fructose, Glucose und Saccharose bekannt, das zu einer verbesserten Mannit-Ausbeute führt. Das Verfahren ist dadurch gekennzeichnet, daß die Hydrierung in Gegenwart eines Raney-Nickeloder Nickel-Katalysators unter Verwendung eines Zusatzes wie einer Zink- oder Magnesiumverbindung durchgeführt wird.

Die GB 801,732 A beschreibt ein Verfahren zur Herstellung eines Polyols, wobei ein entsprechender Zucker unter einem Druck von etwa 15 bis 25 kg/cm² und in Gegenwart einer wäßrigen Lösung eines oder mehrerer aliphatischer Alkohole mit 1 bis 3 Kohlenstoffatomen durchgeführt wird. Die Hydrierung erfolgt unter Verwendung eines Nickel- oder Raney-Nickel-Katalysators.

In Fällen, in denen bei der Hydrierung aus einem Edukt, wie zum Beispiel Isomaltulose, stereoisomere Produkte (Epimere) entstehen können, ist es häufig aus technologischen oder anwendungstechnischen Gründen wünschenswert, die Stereoselektivität der Reaktion so einzustellen, daß ein bestimmtes Verhältnis der Produkte zueinander erhalten wird. Die Entwicklung von Verfahren, die zu einer bestimmten, bisher nicht erhältlichen Produktzusammensetzung führen, ist daher insbesondere für anwendungstechnische Zwecke wünschenswert. Zudem sind die bekannten Verfahren hinsichtlich ihrer Verfahrensführung, der Handhabbarkeit der verwendeten Katalysatoren und der Verfahrenskosten verbesserungsfähig.

Das der vorliegenden Erfindung zugrundeliegende technische Problem besteht also darin, ein Verfahren zur Hydrierung von Zuckern aus der Gruppe bestehend aus Isomaltulose, Leucrose, Trehalulose, Maltulose und Lactulose zu Zuckeralkoholen bereitzustellen, das die vorgenannten Nachteile überwindet und zu bisher nicht erhältlichen Produktzusammensetzungen führt.

Das der Erfindung zugrundeliegende technische Problem wird durch die Bereitstellung eines Verfahrens gemäß Hauptanspruch gelöst. Die Erfindung sieht also insbesondere vor, ein Verfahren zur Hydrierung von Zuckern oder Zuckergemischen aus der Gruppe bestehend aus Isomaltulose, Leucrose, Trehalulose, Lactulose und Maltulose zu Mannit-Epimeren angereicherten Zuckeralkoholgemischen bereitzustellen, wobei die Zucker oder Zuckergemische in wässriger Lösung unter einer erhöhten Temperatur von 60°C bis 150°C und einem erhöhten Druck von 50 bar bis 450 bar mit Wasserstoff unter Verwendung eines trägergebundenen Katalysators hydriert werden und wobei die katalytisch aktive Komponente des Katalysators ein Gemisch aus Nickel, Wolframoxid und gegebenenfalls Nickeloxid umfaßt. Derartige Katalysatoren sind aus der US 3,965,199 bekannt, auf die hinsichtlich der Zusammensetzung und der Herstellungsweise des erfindungsgemäß verwendeten Katalysators verwiesen wird. In besonders bevorzugter Weise wird der Katalysator in extrudierter Form verwendet, wie kommerziell erhältlich.

Im Zusammenhang mit der vorliegenden Erfindung wird unter einem trägergebundenen Nickel-haltigen Katalysator ein Katalysator verstanden, dessen katalytisch aktive Komponente nicht mittels alkalischer Aktivierung von Aluminiumlegierungen mit anderen Metallen präpariert wird, noch Aluminiumlegierungen, auch nicht in Resten, enthält. Der erfindungsgemäß verwendete Katalysator ist also kein Raney-Nickel-Katalysator.

In besonders vorteilhafter Weise ermöglicht die erfindungsgemäße Vorgehensweise in Fällen, in denen aus einem Edukt unterschiedliche stereoisomere Produkte aus der Hydrierungsreaktion hervorgehen, eine von der herkömmlicherweise erhaltenen Produktzusammensetzung abweichende Zusammensetzung zu erhalten. Die Erfindung sieht in einer besonders vorteilhaften Ausgestaltung vor, Isomaltulose zu hydrieren, wobei sich eine von der nach dem Stand der Technik zu erwartenden Produktzusammensetzung von ca. 50 Gew.-% 1,1-GPM zu 50 Gew.-% 1,6-GPS abweichende Zusammensetzung ergibt, insbesondere eine Zusammensetzung mit einem erhöhten 1,1-GPM und einem verminderten 1,6-GPS Anteil im Produkt. Die Erfindung kann in vorteilhafter Weise auch zur Hydrierung von Trehalulose zu 1,1-GPM und 1-O-α-D-Glucopyranosyl-D-sorbit (1,1-GPS), Lactulose zu Lactit sowie 3-0-β-D-Galactopyranosyl-D-mannit, Maltulose zu 3-0-α-D-Glucopyranosyl-D-mannit sowie 4-0-α-D-Glucopyranosyl-D-sorbit (Maltit) und Leucrose zu 5-0-α-D-Glucopyranosyl-D-sorbit (1,5-GPS) sowie 2-0-α-D-Glucopyranosyl-D-mannit (1,2-GPM) eingesetzt werden. Hierbei ergeben sich insbesondere im Fall von Trehalulose ein vergleichsweise erhöhter 1,1-GPM-Anteil, im Fall von Maltulose ein vergleichsweise erhöhter 3-0-α-D-Glucopyranosyl-Dmannit-Anteil und im Fall von Lactulose ein erhöhter 3-0-β-D-Galactopyranosyl-D-mannit-Anteil im Produkt. Die Erfindung führt also bei der Hydrierung der genannten glycopyranosylsubstituierten Ketosen zu einem erhöhten Anteil der Mannit-Epimeren und einem verminderten Anteil der Sorbit-Epimeren im Produkt.

Als Mannit- und Sorbit-Epimeren werden die Zuckeralkohol-Stereoisomeren bezeichnet, die bei der Hydrierung des prochiralen Carbonyl-Kohlenstoffatoms der Ketose beziehungsweise glycosylsubstituierten Fructose (Isomaltulose, Trehalulose usw.) entstehen. Im Zusammenhang mit der vorliegenden Erfindung wird unter einem Mannit-Epimer ein Epimer verstanden, das die Konstitution der Polyolkette der glycosylsubstituierten D-Mannose aufweist. Unter einem Sorbit-Epimer wird ein Epimer verstanden, das die Konstitution der Polyolkette der glycosylsubstituierten D-Glucose aufweist.

Selbstverständlich ist es erfindungsgemäß möglich und besonders bevorzugt, Zuckergemische, wie zum Beispiel ein auch in der EP 0 625 578 B1 beschriebenes Gemisch aus Isomaltulose und Trehalulose sowie gegebenenfalls Glucose, Fructose, Isomelezitose, Rest-Saccharose, Isomaltose und Oligosacchariden zu hydrieren. Auch hier führt die Erfindung zu einem erhöhten Anteil der Mannit-Epimeren im Produkt.

Die Erfindung sieht in besonders vorteilhafter Weise eine Zusammensetzung des erfindungsgemäß verwendeten Katalysators von 5 - 50 Gew.-% Nickel oder Nickeloxid und 0,5 - 16 Gew.-% Wolframoxid, bezogen auf das Gesamtgewicht des Katalysators einschließlich Trägermaterial vor.

In einer weiteren, besonders bevorzugten Ausführungsform weist der Katalysator eine Dichte von 0,60 bis 0,70 kg/l auf.

Die Erfindung sieht vor, daß der Katalysator auf einem Träger gebunden ist. Dieser Träger kann beispielsweise Siliciumdioxid, Kieselgur, TiO₂, SiO₂ · Al₂O₃, Tone, Zeolith, ZrO₂, Silikat oder Aluminiumoxid (Al₂O₃) umfassen.

Die erfindungsgemäß zu verwendenden Katalysatoren können neben Nickel, Nickeloxid und Wolframoxid, insbesondere Wolframtrioxid, bezogen auf einen trägerfreien Formkörper, bis zu 20 Gew.-%, vorzugsweise 15 Gew.-%, anderer Metalle enthalten. Diese Metalle brauchen keine katalytische Aktivität aufweisen und umfassen beispielsweise Eisen, Mangan, Titan, Aluminium oder Silicium.

Die erfindungsgemäß einzusetzenden Katalysatoren werden durch Niederschlagung beziehungsweise Adsorption der katalytisch aktiven Komponenten, das heißt, Nickel und Wolframoxid sowie gegebenenfalls Nickeloxid, aus einer Lösung auf dem inerten Trägermaterial hergestellt. Der auf diese Weise hergestellte Katalysator-Vorläufer kann nach Aktivierung mit Wasserstoff direkt zur Hydrierung eingesetzt werden. Der Katalysator-Vorläufer kann erfindungsgemäß jedoch weiterbehandelt werden, vorzugsweise einem Extrusionsverfahren unterzogen werden. Die Erfindung sieht jedoch auch vor, daß der Katalysator-Vorläufer durch Verpressen des wie beschrieben imprägnierten Trägermaterials unter hohem Druck hergestellt wird, wobei gegebenenfalls zur Verbesserung des Haftvermögens der Partikel auch Graphit und/oder Klebstoff in Mengen unter 1 Gew.-%, bezogen auf das Katalysatorgewicht, eingesetzt werden können. Die Förmlinge werden anschließend calciniert und mit Wasserstoff reduziert, wobei sich bei letzterem Schritt die katalytisch aktiven Metallkristallite ausbilden. Der Katalysator weist in stabilisierter Form eine inertisierende Adsorptionsschicht an seiner Oberfläche auf und wird erst durch Wasserstoffbegasung aktiviert, indem die monomolekulare Adsorptionsschicht entfernt beziehungsweise die Oxidschicht reduziert und damit der Katalysator mit Wasserstoff aktiviert wird.

Die Katalysatoren können in Form von Kugeln, Tabletten, Granulaten, Stäbchen, jeweils mit oder ohne Bohrungen, ausgeführt sein. Selbstverständlich ist es auch möglich, die Katalysatoren als Pulverkatalysatoren, beispielsweise im Suspensionsverfahren, einzusetzen.

Das erfindungsgemäße Verfahren verwendet als Edukt Lactulose, Trehalulose, Maltulose, Isomaltulose, Leucrose oder Gemische davon. Die Zucker können flüssig eingesetzt werden. Das Edukt wird gemäß einer besonders bevorzugten Ausführungsform der Erfindung in entmineralisiertem Wasser gelöst, wobei eine 10 bis 70 gew.-%ige, insbesondere 15 bis 50 gew.-%ige, bevorzugt 40 gew.-%ige Lösung (bezogen auf Trockensubstanz) eingestellt wird. Der pH-Wert liegt bevorzugt in einem Bereich von 3,0 bis 12,0. Der pH-Wert kann beispielsweise durch Zugabe von wasserlöslichen, basisch reagierenden Verbindungen, wie Alkalicarbonaten oder Ammoniak in wässriger Lösung, oder sauer reagierenden Verbindungen, wie Zuckersäuren, Sorbinsäure oder Zitronensäure, auf den gewünschten pH-Wert eingestellt werden.

Das erfindungsgemäße Verfahren sieht vor, die Hydrierung mit reinem Wasserstoff durchzuführen, der auf einen Druck von 50 bis 450 bar, bevorzugt 150 bis 300 bar, vorkomprimiert wird. Die Hydrierung kann in besonders bevorzugter Weise kontinuierlich im Festbettverfahren durchgeführt werden. Dabei kann in bekannter Weise ein Gleichstrom- oder ein Gegenstromverfahren eingesetzt werden. Die Erfindung sieht jedoch auch vor, daß die Hydrierung diskontinuierlich im Suspensionsverfahren mit dem Pulverkatalysator oder pulverisiertem Festbettkatalysator durchgeführt wird.

Das erfindungsgemäße Verfahren wird vorzugsweise in einem beispielsweise als Hochdruckrohr aus Stahl ausgeführten Hydrierreaktor durchgeführt, wobei der Hydrierreaktor ganz oder teilweise mit dem trägergebundenen Katalysator gefüllt ist. Es kann auch vorgesehen sein, den Katalysator in einem Katalysatorkorb anzuordnen. Die Erfindung umfaßt selbstverständlich auch die Verwendung von Hydrierreaktoren, die beispielsweise aus verschiedenen Einzelreaktoren aufgebaut sind. In einer besonders bevorzugten Ausführungsform sieht die Erfindung vor, in dem Hydrierreaktor Rührvorrichtungen vorzusehen, mit Hilfe derer die Edukte und das Hydriergas besser miteinander in Kontakt gebracht werden können.

Die Hydrierung wird vorzugsweise bei Temperaturen von 60°C bis 150°C, vorzugsweise von 70°C bis 120°C, vorgenommen.

Das erfindungsgemäße Verfahren führt zur Gewinnung von Zuckeralkoholen oder Zuckeralkoholgemischen in einer Reinheit von bis über 99 Gew.-%, bezogen auf die Trockenmasse. Es ist möglich, den Gehalt an nicht umgesetzten Zuckern oder Zuckergemischen auf Werte von 0,2 Gew.-% oder weniger zu senken.

Die Erfindung wird anhand der folgenden Figuren und der dazugehörigen Ausführungsbeispiele näher erläutert. Es zeigen:
- Figur 1: einen erfindungsgemäß verwendeten Hydrierreaktor in schematischer Darstellung;
- Figur 2: eine graphische Darstellung der Produktbildung in Abhängigkeit von der Zeit zum Beispiel 1.

### Beispiel 1:

### Hydrierung von Isomaltulose mittels des erfindungsgemäßen Verfahrens

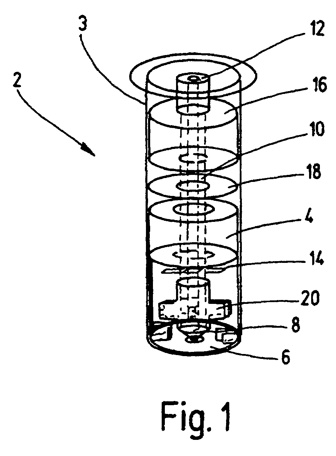

In einem 750 ml Innenvolumen aufweisenden Hydrierreaktor 2 gemäß Figur 1 wurde im diskontinuierlichen Festbettverfahren Isomaltulose hydriert. Der Hydrierreaktor 2 enthält in einem Edelstahlrohr einen Katalysatorkorb 4 (Volumen: 138,9 cm³) mit einem Innenvolumen von 138,9 cm³. Im Katalysatorkorb 4 befinden sich 146,77 g (feucht) beziehungsweise 70,27 g (trocken) Nickel, Nickeloxid und Wolframoxid umfassender Katalysator (Südchemie T-4190 RS 3 mm).

In bevorzugter Weise wird das katalytisch aktive Material (Nickel, Nickeloxid, Wolframoxid) zunächst aus löslicher Form auf dem feindispersen und dispergierten Trägermaterial (Träger: Alumosilikat) durch Fällung und Adsorption abgeschieden. Anschließend erfolgt eine Vermengung mit Bindern, Porenbildnern und weiteren Komponenten. Das homogene Gemisch wird zu Förmlingen extrudiert, getrocknet und calciniert. Zusätzlich können Schmiermittel, Porenbildner, Verformungshilfsstoffe, Plastifizierer etc., die zur Formausbildung zugesetzt werden, enthalten sein. Die Formkörper werden gegebenenfalls zwischen 80 und 120°C getrocknet. Anschließend wird eine Calcinierung bei Temperaturen unter 850°C, vorzugsweise zwischen 500 und 700°C, durchgeführt. Bei der Calcination erfahren die Extrudate eine Härtung, und das Makro- und Mesoporensystem bildet sich aus. In einer Aktivierungsstufe erfolgt anschließend eine Reduktion der oxidischen Verbindungen auf der Trägeroberfläche (Nickeloxid, Wolframoxid) mittels Wasserstoff bei Temperaturen zwischen 300 und 600°C. Um den Katalysator an der Luft lagerfähig zu machen, wird mit O₂/CO-Mischungen eine Oberflächeninertisierung durchgeführt (Stabilisierung). Dabei wird die aktive Fläche mit einer dünnen, monomolekularen Adsorptionsschicht belegt.

Der Hydrierreaktor 2 weist an seiner im Bodenbereich angeordneten Zentrierscheibe 6 ein unteres Wellenlager 8 auf. Das untere Wellenlager 8 sowie ein oberes Wellenlager 12 dienen der Lagerung einer Edelstahl-Rührwelle 10, die Rührflügel 14 trägt. Die Rührwelle 10 wird elektromagnetisch mittels des Rührmagneten 16 angetrieben. Die Figur 1 stellt ferner Strömungsunterbrecher 18 sowie einen Gasverteilungsflügel 20 dar.

In den Hydrierreaktor 2 werden 500 ml wässrige Eduktlösung, hier Isomaltulose-Lösung (0,1 % Fructose, 0,1 % Glucose, 98 % Isomaltulose, 1,2 % Trehalulose, 0,3 % Isomaltose, 0,3 % Rest, Angaben in Gew.-%) (30 Gew.-% Trockensubstanz) (pH: 5,3), eingefüllt, über den Gasverteilungsflügel 20 Wasserstoff mit einem Druck von 150 bar und einer Rotationsgeschwindigkeit der Rührwelle 10 von 600 Umdrehungen pro Minute eingeleitet und eine Temperatur von 70° C eingehalten. Zu Beginn der Reaktion, nach 2, 3, 4, 5, 6 und 22 Stunden werden Proben der Reaktionslösung (pH: 6,5) entnommen und auf ihren Gehalt an Isomaltulose, 1,1-GPM, 1,6-GPS, Mannit, Sorbit und Restsaccharid bestimmt.

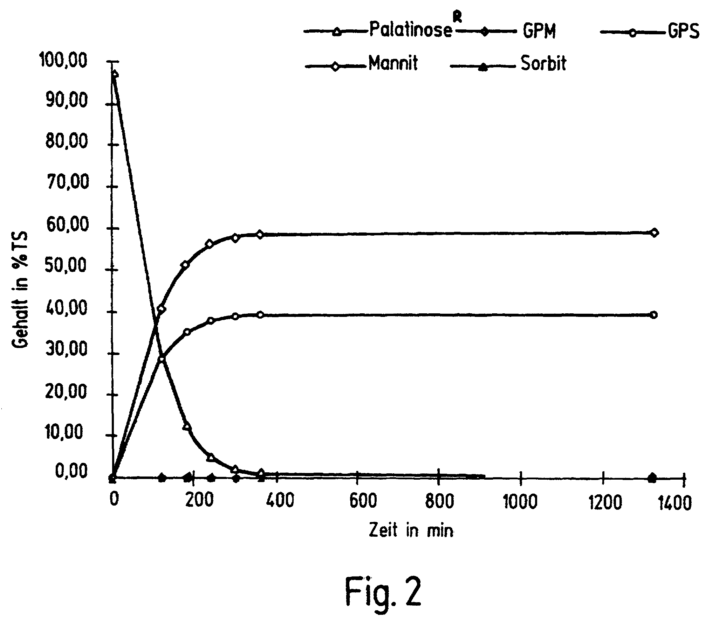

Die Ergebnisse sind in der Tabelle 1 und grafisch in der Figur 2 dargestellt.

**Tabelle 1**

| Versuchsdauer in min | Isomaltulose % | 1,1-GPM % | 1,6-GPS % | Mannit % | Sorbit % | Rest % |
|---|---|---|---|---|---|---|
| 0 | 98,00 | 0,00 | 0,00 | 0,00 | 0,00 | 2,00 |
| 120 | 29,49 | 40,96 | 28,65 | 0,03 | 0,03 | 0,84 |
| 180 | 12,56 | 51,60 | 35,22 | 0,03 | 0,05 | 0,54 |
| 240 | 4,64 | 56,53 | 38,12 | 0,04 | 0,05 | 0,62 |
| 300 | 1,93 | 58,20 | 39,20 | 0,05 | 0,07 | 0,55 |
| 360 | 1,02 | 58,81 | 39,52 | 0,05 | 0,06 | 0,54 |
| 1320 | 0,11 | 59,45 | 39,92 | 0,05 | 0,08 | 0,39 |

Erfindungsgemäß führt die Hydrierung zu einer von dem erwarteten 50:50 Verhältnis von 1,1-GPM zu 1,6-GPS abweichenden Produktzusammensetzung. Es wird vergleichsweise mehr Mannit-Epimer und weniger Sorbit-Epimer gebildet.

### Beispiel 2:

### Hydrierung von Isomaltulose mittels des erfindungsgemäßen Verfahrens

Die Verfahrensbedingungen und die Verfahrensapparatur entsprechen den in Beispiel 1 genannten. Es wurde jedoch nur eine Probe nach 22 Stunden entnommen. Das verwendete Edukt weist die in Tabelle 2 gezeigte Zusammensetzung auf (%-Angaben sind im folgenden, falls nicht anders angegeben, als Gew.-% zu verstehen):

**Tabelle 2**

| Nr. | Edukt | Gehalt |
|---|---|---|
| 1 | Isomaltulose | 98,50 % a. TS |
| 2 | Trehalulose | 1,13 % a. TS |
| 3 | Isomaltose | 0,23 % a. TS |
| 4 | Isomelezitose | 0,08 % a. TS |
| 5 | Restsaccharide | 0,06 % a. TS |

Die Tabelle 3 verdeutlicht, daß das erfindungsgemäße Verfahren zu einer anderen Produktzusammensetzung führt, als ein ansonsten gleiches Verfahren, das jedoch mit einem Vergleichskatalysator durchgeführt wurde. Für das Vergleichsverfahren wurde ein trägerfreier Raney-Nickel-Katalysator verwendet, der durch Tablettierung eines aktivierten Nickelpulvers hergestellt wurde. Die Tabletten weisen eine Zylinderhöhe von 5 mm und einen Durchmesser von 5 mm bei einer Druckfestigkeit von 147 N und einer inneren Oberfläche von 33 m²/g auf.

**Tabelle 3**

| Nr. | Produkt | Vergleichsverfahren | Erfindung |
|---|---|---|---|
| 1 | 1,1-GPM | 49,09 % a. TS | 58,34 % a. TS |
| 2 | 1,6-GPS | 49,45 % a. TS | 40,83 % a. TS |
| 3 | GPI | 0,33 % a. TS | 0,07 % a. TS |
| 4 | Mannit | 0,05 % a. TS | 0,05 % a. TS |
| 5 | Sorbit | 0,11 % a. TS | 0,09 % a. TS |
| 6 | hydrierte und nicht-hydrierte Restsaccharide | 0,97 % a. TS | 0,61 % a. TS |

Das Vergleichsverfahren führt zu einem ca. 1:1 Verhältnis von 1,1-GPM und 1,6-GPS, während das erfindungsgemäße Verfahren zu einem erhöhten 1,1-GPMund einem verminderten 1,6-GPS-Anteil im Produkt führt.

### Beispiel 3:

### Hydrierung eines Gemisches aus Isomaltulose und Trehalulose (bekannt aus EP 0 625 578 B1)

Die Verfahrensführung und die Verfahrensapparatur entsprechen den in Beispiel 1 genannten. Das Vergleichsverfahren verwendet einen Katalysator gemäß dem in Beispiel 2 beschriebenen.

Das eingesetzte Edukt stellt ein Zuckergemisch der in Tabelle 4 gezeigten Zusammensetzung dar:

**Tabelle 4**

| Nr. | Edukt | Gehalt |
|---|---|---|
| 1 | Glucose | 3,64 % a. TS |
| 2 | Fructose | 2,50 % a. TS |
| 3 | Isomaltulose | 84,02 % a. TS |
| 4 | Trehalulose | 7,64 % a. TS |
| 5 | Isomaltose | 1,39 % a. TS |
| 6 | Isomelezitose | 0,38 % a. TS |
| 7 | Restsaccharide | 0,43 % a. TS |

Die Hydrierung gemäß der vorliegenden Erfindung und gemäß des Vergleichsverfahrens führt zu folgendem Ergebnis:

**Tabelle 5**

| Nr. | Produkt | Vergleichsverfahren | Erfindung |
|---|---|---|---|
| 1 | 1,1-GPM | 46,51 % a. TS | 54,78 % a TS |
| 2 | 1,6-GPS + 1,1-GPS | 46,47 % a. TS | 38,29 % a.TS |
| 3 | Mannit | 1,59 % a. TS | 1,22 % a.TS |
| 4 | Sorbit | 3,78 % a. TS | 3,70 % a.TS |
| 5 | hydrierte und nicht-hydrierte Restsaccharide | 1,65 % a. TS | 2,01 % a.TS |

Das Vergleichsverfahren führt zu einem 1:1 Verhältnis von 1,1-GPM zu 1,1-GPS/1,6-GPS, während das erfindungsgemäße Verfahren zu einem erhöhten 1,1-GPM und einem verminderten 1,1-GPS/1,6-GPS Anteil im Produkt führt.

### Beispiel 4:

### Hydrierung von Lactulose mittels des erfindungsgemäßen Verfahrens

Die Verfahrensführung und die Verfahrensapparatur entsprechen den in Beispiel 1 genannten. Das Vergleichsverfahren verwendet einen Katalysator gemäß dem in Beispiel 2 beschriebenen.

Die Hydrierung von Lactulose (99,48 Gew.-%, 0,52 Gew.-% Restsaccharide, bezogen auf Trockensubstanz) gemäß der vorliegenden Erfindung und gemäß des Standes der Technik führt zu folgendem Ergebnis:

**Tabelle 6**

| Nr. | Produkt | Vergleichsverfahren | Erfindung |
|---|---|---|---|
| 1 | 1,3-GalPM | 46.38 % a. TS | 64,29 % a.TS |
| 2 | 1,4-GalPS (Lactit) | 51,60 % a. TS | 34,73 % a.TS |
| 3 | Mannit | 0,38 % a. TS | 0,08 % a.TS |
| 4 | Sorbit | 0,04 % a. TS | 0,09 % a.TS |
| 5 | Galactit | 0,91 % a. TS | 0,64 % a.TS |
| 6 | hydrierte und nicht-hydrierte Restsaccharide | 0,69 % a. TS | 0,17 % a.TS |

Das Verfahren gemäß.der Erfindung führt zu einem vergleichsweise erhöhten 1,3-GalPM- und einem vergleichsweise verminderten 1,4-GalPS (Lactit)-Anteil im Produkt.

### Beispiel 5:

### Hydrierung von Trehalulose

Die Verfahrensführung und die Verfahrensapparatur entsprechen den in Beispiel 1 genannten. Das Vergleichsverfahren verwendet einen Katalysator gemäß dem in Beispiel 2 beschriebenen.

Die Hydrierung von Trehalulose (94,82 %, 2,50 % Isomaltulose, 2,68 % Restsaccharide, Gew.-% bezogen auf Trockensubstanz) gemäß der vorliegenden Erfindung und des Vergleichsverfahrens führt zu folgendem Ergebnis:

**Tabelle 7**

| Nr. | Produkt | Vergleichsverfahren | Erfindung |
|---|---|---|---|
| 1 | 1,1-GPM | 53,29 % a. TS | 78,95 % a. TS |
| 2 | 1,1-GPS + 1,6-GPS | 41,10 % a. TS | 15,46 % a. TS |
| 3 | Mannit | 0,02 % a. TS | 0,39 % a. TS |
| 4 | Sorbit | 1,02 % a. TS | 1,47 % a. TS |
| 5 | hydrierte und nicht-hydrierte Restsaccharide | 4,57 % a. TS | 3,73 % a. TS |

Mit dem erfindungsgemäßen Verfahren wird ein wesentlich erhöhter 1,1-GPM-Anteil im Produkt als mit dem herkömmlichen Verfahren erzielt. Dementsprechend ist der 1,1-GPS-Anteil erheblich reduziert.

1,6-GPS stammt aus in Resten eingesetzter Isomaltulose im Edukt.

### Beispiel 6:

### Hydrierung von Maltulose

Die Verfahrensführung und die Verfahrensapparatur entsprechen den in Beispiel 1 genannten. Das Vergleichsverfahren verwendet einen Katalysator gemäß dem in Beispiel 2 beschriebenen.

Die Zusammensetzung des Edukts war wie folgt:

**Tabelle 8**

| Nr. | Edukt | Gehalt |
|---|---|---|
| 1 | Maltulose | 83,43 % a. TS |
| 2 | Fructose | 5,74 % a. TS |
| 3 | Glucose | 3,87 % a. TS |
| 4 | Restsaccharide | 6,96 % a. TS |

Die Hydrierung gemäß der vorliegenden Erfindung und gemäß des Vergleichsverfahrens führt zu folgendem Ergebnis:

**Tabelle 9**

| Nr. | Produkt | Vergleichsverfahren | Erfindung |
|---|---|---|---|
| 1 | 1,3-GPM | 37,29 % a. TS | 44,78 % a. TS |
| 2 | 1,4-GPS (Maltit) | 41,49 % a. TS | 29,23 % a. TS |
| 3 | Sorbit | 7,36 % a. TS | 15,15 % a. TS |
| 4 | Mannit | 5,44 % a. TS | 8,02 % a. TS |
| 5 | hydrierte und nicht-hydrierte Restsaccharide | 7,97 % a. TS | 2,82 % a. TS |

Es wird ein vergleichweise höherer Anteil an Mannit-Epimeren als an Sorbit-Epimeren im Produkt erhalten.

### Beispiel 7:

### Hydrierung von Zuckern mit Pulver-Katalysatoren (Slurry-Verfahren, diskontinuierliches Verfahren)

### (1) Autoklavenanlage und Versuchsparameter:

- 750 mL Laborautoklav mit induktiv betriebenem Rührwerk
- Reaktionstemperatur : 70°C
- Wasserstoffdruck : 150 bar
- Rührerdrehzahl : 600 Upm
- Zuckerlösung : 500 mL mit 30 % TS
- Katalysatormenge : ca. 25 g (feucht)
- Reaktionszeit : 22 h

### (2) Ansatz :

Die Reaktionslösung (500 mL, 30 % TS) wird in den vorthermostatisierten Autoklaven nach Figur 1, allerdings ohne Korbeinsatz mit modifiziertem Rührwerk, gegeben. Anschließend werden 25 g Katalysatorpulver (Katalysatorzusammensetzung entspricht der in Beispiel 1 genannten) beigefügt und dann (nicht vorher) die Rührachse eingesetzt. Nach 3maliger Inertisierung der Anlage mit Stickstoff erfolgt die Hydrierung bei 150 bar und 70°C Reaktionstemperatur. Nach 22 h wird unter Druck auf Raumtemperatur abgekühlt, entspannt und nach Inertisierung der Anlage die Produktlösung entnommen und der Katalysator abfiltriert.

### Beispiel 8:

### Hydrierung von Isomaltulose mittels eines Pulverkatalysators

Die Verfahrensführung und die Verfahrensapparatur entsprechen den in Beispiel 7 genannten. Das Edukt weist folgende Zusammensetzung auf:

**Tabelle 10**

| Nr. | Edukt | Gehalt |
|---|---|---|
| 1 | Fructose | 0,15 % a. TS |
| 2 | Glucose | 0,12 % a. TS |
| 3 | Isomaltulose | 98,21 % a. TS |
| 4 | Trehalulose | 1,30 % a. TS |
| 5 | Isomaltose | 0,10 % a. TS |
| 6 | Rest | 0,12 % a. TS |

Die Hydrierung gemäß der vorliegenden Erfindung führt zu folgendem Ergebnis:

**Tabelle 11**

| Nr. | Produkt | Erfindung |
|---|---|---|
| 1 | 1,1-GPM | 52,29 % a. TS |
| 2 | 1,6-GPS | 46,74 % a. TS |
| 3 | Mannit | 0,07 % a. TS |
| 4 | Sorbit | 0,13 % a. TS |
| 5 | hydrierte und nicht-hydrierte Restsaccharide | 0,77 % a. TS |

Es wird ein vergleichsweise (1:1 Verhältnis von 1,1-GPM zu 1,6-GPS erwartet) erhöhter 1,1-GPM- und verminderter 1,6-GPS-Gehalt erzielt.

### Beispiel 9:

### Hydrierung von Trehalulose mittels eines Pulverkatalysators

Die Verfahrensführung und die Verfahrensapparatur entsprechen den in Beispiel 7 genannten. Das Edukt weist folgende Zusammensetzung auf:

**Tabelle 12**

| Nr. | Edukt | Gehalt |
|---|---|---|
| 1 | Fructose | 0,16 % a. TS |
| 2 | Glucose | 2,59 % a. TS |
| 3 | Isomaltulose | 2,61 % a. TS |
| 4 | Trehalulose | 91,80 % a. TS |
| 5 | Restsaccharide | 2,84 % a. TS |

Die Hydrierung gemäß der vorliegenden Erfindung führt zu folgendem Ergebnis:

**Tabelle 13**

| Nr. | Produkt | Erfindung |
|---|---|---|
| 1 | 1,1-GPM | 60,82 % a. TS |
| 2 | 1,1-GPS + 1,6-GPS | 32,17 % a. TS |
| 3 | Mannit | 0,10 % a. TS |
| 4 | Sorbit | 1,16 % a. TS |
| 5 | hydrierte und nicht-hydrierte Restsaccharide | 5,75 % a. TS |

Es wird ein-erhöhter 1,1-GPM-Anteil und eine reduzierter 1,1-GPS-Anteil im Produkt erhalten. Das 1,6-GPS stammt aus der im Edukt in Resten enthaltenen Isomaltulose.

## Patentansprüche

1. Verfahren zur Hydrierung von Zuckern oder Zuckergemischen aus der Gruppe bestehend aus Isomaltulose, Trehalulose, Maltulose, Leucrose und Lactulose zu Mannit-Epimeren angereicherten Zuckeralkoholgemischen, wobei die Zucker oder Zuckergemische in wässriger Lösung unter einer erhöhten Temperatur von 60°C bis 150°C und einem erhöhten Druck von 50 bar bis 450 bar mit Wasserstoff unter Verwendung eines trägergebundenen Nickel-, Nickeloxid- und Wolframoxid-haltigen Katalysators hydriert werden.

2. Verfahren nach Anspruch 1, **dadurch gekennzeichnet, daß** der Katalysator eine Dichte von 0,60 bis 0,70 kg/l aufweist.

3. Verfahren nach einem der vorhergehenden Ansprüche, **dadurch gekennzeichnet, dass** der Katalysator 5 bis 45 Gew.-% Nickel und 0,5 bis 16 Gew.-% Wolframoxid, bezogen auf das Gesamtgewicht des Katalysators einschließlich Träger, enthält.

4. Verfahren nach einem der vorhergehenden Ansprüche, **dadurch gekennzeichnet, daß** der Träger Siliciumdioxid und Aluminiumoxid umfaßt.

5. Verfahren nach einem der vorhergehenden Ansprüche, **dadurch gekennzeichnet, daß** der Katalysator im Extrusionsverfahren hergestellt ist.

6. Verfahren nach einem der vorhergehenden Ansprüche, **dadurch gekennzeichnet, daß** die Hydrierung kontinuierlich, halb-kontinuierlich oder diskontinuierlich durchgeführt wird.

7. Verfahren nach einem der vorhergehenden Ansprüche, **dadurch gekennzeichnet, dass** die Hydrierung im Festbett- oder Suspensionsverfahren durchgeführt wird.

8. Verfahren nach einem der vorhergehenden Ansprüche, **dadurch gekennzeichnet, dass** die erhöhte Temperatur 70°C beträgt.

9. Verfahren nach einem der vorhergehenden Ansprüche, **dadurch gekennzeichnet, dass** die Eduktkonzentration in der Ausgangslösung auf 10 bis 70 Gew.-% eingestellt ist.

10. Verfahren nach Anspruch 10, **dadurch gekennzeichnet, dass** die Eduktkonzentration in der Ausgangslösung auf 15 bis 50 Gew.-% eingestellt ist.

11. Verfahren nach Anspruch 11, **dadurch gekennzeichnet, dass** die Eduktkonzentration in der Ausgangslösung auf 40 Gew.-% eingestellt ist.

## Claims

1. Method for hydrogenating sugars or sugar mixtures from the group comprising isomaltulose, trehalulose, maltulose, leucrose and lactulose to form sugar alcohol mixtures enriched with mannite epimers, wherein the sugar or sugar mixtures are hydrogenated in aqueous solution at a raised temperature of between 60°C and 150°C and a raised pressure of between 50 bar and 450 bar with hydrogen using a carrier-bonded catalyst containing nickel, nickel oxide and tungsten oxide.

2. Method according to claim 1, **characterised in that** the catalyst has a density of between 0.60 and 0.70 kg/l.

3. Method according to one of the preceding claims, **characterised in that** the catalyst contains between 5 and 45 wt-% nickel and between 0.5. and 16 wt-% tungsten oxide in relation to the total weight of the catalyst including the carrier.

4. Method according to one of the preceding claims, **characterised in that** the carrier includes silicon dioxide and aluminium oxide.

5. Method according to one of the preceding claims, **characterised in that** the catalyst is produced by an extrusion method.

6. Method according to one of the preceding claims, **characterised in that** the hydrogenation is carried out continuously, semi-continuously or discontinuously.

7. Method according to one of the preceding claims, **characterised in that** the hydrogenation is carried out by means of a fixed bed or suspension method.

8. Method according to one of the preceding claims, **characterised in that** the raised temperature is 70°C.

9. Method according to one of the preceding claims, **characterised in that** the educt concentration in the initial solution is set at between 10 and 70 wt-%.

10. Method according to claim 9, **characterised in that** the educt concentration in the initial solution is set at between 15 and 50 wt-%.

11. Method according to claim 10, **characterised in that** the educt concentration in the initial solution is set at 40 wt-%.

## Revendications

1. Procédé d'hydrogénation de sucres ou de mélanges de sucres, choisis dans le groupe formé de l'isomaltulose, du trehalulose, du maltulose, du leucrose et du lactulose, en mélanges d'alcools de sucres enrichis en épimères de mannitol, dans lequel les sucres ou les mélanges de sucres sont hydrogénés en solution aqueuse, sous une température augmentée allant de 60°C à 150°C, et une pression augmentée allant de 50 bars à 450 bars, avec de l'hydrogène en utilisant un catalyseur lié à un support contenant du nickel, de l'oxyde de nickel et de l'oxyde de tungstène.

2. Procédé selon la revendication 1,
**caractérisé en ce que**
le catalyseur possède une densité de 0,60 à 0,70 kg/l.

3. Procédé selon l'une des revendications précédentes,
**caractérisé en ce que**
le catalyseur contient de 5 à 45 % en poids de nickel et de 0,5 à 16 % en poids d'oxyde de tungstène rapporté au poids total de catalyseur y compris le support.

4. Procédé selon l'une des revendications précédentes,
**caractérisé en ce que**
le support comprend du dioxyde de silicium et de l'oxyde d'aluminium.

5. Procédé selon l'une des revendications précédentes,
**caractérisé en ce que**
le catalyseur est produit dans un procédé par extrusion.

6. Procédé selon l'une des revendications précédentes,
**caractérisé en ce que**
l'hydrogénation est effectuée en continu, en semi-continu ou d'une manière discontinue.

7. Procédé selon l'une des revendications précédentes,
**caractérisé en ce que**
l'hydrogénation est effectuée selon un procédé en lit fixe ou en suspension.

8. Procédé selon l'une des revendications précédentes,
**caractérisé en ce que**
la température accrue s'élève à 70°C.

9. Procédé selon l'une des revendications précédentes,
**caractérisé en ce que**
la concentration en éduit dans la solution de départ est ajustée à 10 à 70 % en poids.

10. Procédé selon la revendication 9,
**caractérisé en ce que**
la concentration en éduit dans la solution de départ est ajustée à 15 à 50 % en poids.

11. Procédé selon la revendication 10,
**caractérisé en ce que**
la concentration en éduit dans la solution de départ est ajustée à 40 % en poids.
